# EUROPEAN PATENT APPLICATION

(11) **EP 3 034 088 A1**
(43) Date of publication of application: **22.06.2016**
(21) Application number: 14198577.0
(22) Date of filing: 17.12.2014
(51) Int. Cl.: A61K 36/185, A61K 36/28, A61K 36/738, A61P 3/00, A61P 3/10

(54) **Pharmaceutically active extract, a method for its preparation and the use thereof**

(71) Applicant: RosePharmed, 14667 93143 Tehran (IR)
(72) Inventor: Khorram Khorshid, Hamid Reza, Tehran (IR); Madani, Seyed Hessameddin, Tehran (IR); Farzamfar, Bardia, Tehran (IR); Heshmat, Ramin, Tehran (IR); Gharibdoust, Farhad, Tehran (IR); Madani, Pejman, Tehran (IR); Radmanesh, Ramin, Tehran (IR); Larijani, Bagher, Tehran (IR)
(74) Representative: Scholz, Volker

(57) **Abstract**

The present invention relates to a pharmaceutically active extract of *tanacetum vulgare L., urtica dioica L.* and *rosa cinina,* a method for its preparation and the use thereof.

## Description

The present invention relates to a pharmaceutically active extract, a method for its preparation and its use in the treatment of diabetic neuropathy.

Diabetes mellitus is one of the most important diseases in the world. Near 300 million people will suffer from diabetes in 2025. Long term complications of diabetes are common and responsible for the great amount of disease morbidity and treatment costs. One of the most important complications of diabetes is diabetic neuropathy.

Diabetic neuropathy (DN) is the most common diabetic complication associates with a 45-50% lifetime risk among diabetic patients. Peripheral diabetic neuropathy may be asymptotic in up to 50% of cases and in 15% symptomatic patients, symptoms of neuropathy require treatment. Further, DN is often accompanied with significant morbidity, mortality and economic burden.

The most troublesome and important problem due to neuropathy is food ulcers and subsequent gangrene and food amputation which is responsible for 50-75% of non traumatic lower limb amputation.

Diabetic neuropathy has a multifactorial pathogenesis with different biochemical mechanisms such as increase an oxidative stress, neuro inflammation, decrease in neuronal perfusion and consequently interneuron hypoxia.

Amitriptyline, imipramine, gabapentin and duloxetine are used for symptomatic therapy of peripheral neuropathy. However, these drugs have adverse effects which limit the use for long term therapy or for administration at high doses.

Rudroju et al. Pain Physician 2013, 16(6) 705 discloses comparative efficiency and safety of several antidepressants and anticonvulsants in painful diabetic neuropathy.

Wiffen et al. Cochrane Database Syst. Rev. 2013 provides an overview in regard to antiepileptic drugs for neuropathic pain and fibromyalgia.

Tanenberg et al. Pain Pract. 2013 discloses duloxetine compared with pregabalin for diabetic peripheral neuropathic pain management in patients with suboptimal pain response to gabapentin and treated with or without antidepressants.

Accordingly, it is an object of the present invention to provide a medication for the treatment of diabetic neuropathy which overcomes the drawbacks of the prior art. In particular, a medication shall be provided allowing the effective treatment of the symptoms and signs of diabetic neuropathy and which can be obtained in an easy, cost efficient and less resource consuming way, preferably from natural sources.

This object is achieved by a method for preparing a pharmaceutically active extract compriseing the steps:
a) providing a first plant material of *tanacetum vulgare L.,* a second plant material of *urtica dioica L.,* and a third plant material of *rosa canina*;
b) optionally grinding the first plant material and the second plant material;
c) extracting the first plant material, the second plant material and the third plant material separate from each other by means of an organic solvent to obtain a first extract from the first plant material, a second extract from the second plant material and a third extract from the third plant material, wherein the first extract, the second extract and the third extract can comprise at least parts of the respective plant material;
d) mixing at least part of the first extract, at least part of the second extract and at least part of the third to obtain an extract mixture; and
e) filtrating and optionally concentrating the extract mixture to obtain the pharmaceuticalty active extract.

In a preferred embodiment, the first plant material comprises the aerial parts of *tanacetum vulgare L.*

Preferably, the second plant material comprises the aerial parts of *urtica dioica L.*

Even preferred, the third plant material comprises the fruits of *rosa canina.*

In a further preferred embodiment, the organic solvent is at least one alcohol, preferably is ethanol.

Preferably, the plant material:alcohol ratio in the extracting step c) is 5:1-1:20 by weight, preferably 1:1-1:5 by weight.

It can be preferred that, at least one of the steps a)-e) is carried out in the dark.

Preferred, extracting is carried out in a temperature range from 20-100°C, preferably in a temperature range of 20-60°C, most preferably in a temperature range from 30-50°C.

Further preferred, extracting conducted for 1-200 days, preferably for 7-70 days, most preferably for 10-40 days.

Preferably, concentrating is concentrating to dryness.

The object is also achieved by a pharmaceutically active extract obtained by the method of the present invention.

The object is further achieved by a pharmaceutical composition comprising the inventive pharmaceutically active extract.

In the same way, the object is achieved by the inventive pharmaceutical composition further comprising selenium, urea and/or phytoprotein and/or an antioxidant as well as optionally further excipients such as lubricants, fillers and/or binders.

Finally, the object is achieved by the inventive pharmaceutically active extract and/or the inventive pharmaceutical composition for use in the treatment of diabetic neuropathy and/or the symptoms of diabetic neuropathy.

Preferably, pharmaceutically active extract, wherein the diabetic neuropathy is peripheral diabetic neuropathy.

Surprisingly, it was found that the inventive pharmaceutically active extract can be obtained from natural materials in an easy and cost efficient way. It was further surprisingly found that the inventive extract, can effectively be used for the treatment of diabetic neuropathy, particularly when used in pharmaceutical compositions further comprising additional ingredients to take into account the multifactorial pathogenesis of diabetic neuropathy.

The pharmaceutically active extract should be preferably be present in the above-mentioned pharmaceutical compositions in a concentration of about 0.1 to 99.5, preferably of about 0.5 to 95% by weight of the total mixture. The term "pharmaceutical composition", as used herein, is intended to comprise an extract of the present invention. Also considered is the parmaceutical composition comprising at least one pharmaceutically active component of the extract of the present invention and/or at least one derivative or analog of that active component and corresponding salts thereof.

Further, the pharmaceutical composition can comprise further pharmaceutically active compounds, extracts, mixtures etc.

The pharmaceutical composition can be, for example, in a liquid form, e.g. a solution, siyrup, elixir, emulsion and suspension, or in a solid form, e.g. a capsule, tablet, pill, powder, and suppository. Granules or semisolid forms and gelcaps are also considered. In case that the pharmaceutical composition is a liquid or a powder, the dosage unit optionally is to be measured, e.g. in the dosage unit of a teaspoon full. In addition to the extract or the pharmaceutically active component, the pharmaceutical composition can comprise, for example, flavoring agents, sweeteners, dyes, preservatives, stabilizers, coloring agents, diluents, suspending agents, granulating agents, lubricants, binders and disintegrating agents. A tablet, for example, can be coated.

All of the ingredients of the pharmaceutical composition have to be pharmaceutically acceptable. "Pharmaceutically acceptable" in terms of the present invention means at least non toxic.

The pharmaceutically acceptable carrier may take a wide variety of forms depending upon the desired routes of administration. The term comprises conventional pharmaceutical diluents such as water or ethanol and conventional tabletting ingredients such as corn starch, lactose, sucrose, sorbitol, talc, stearic acid, magnesium stearat, dicalcium phosphate or gums.

Administration of the pharmaceutical composition of the present invention can use different routes, such as oral, sublingual, parenteral, intravenous, intraperitoneal, nasal, vaginal, rectal, subcutaneous, intradermal, intramuscular and topic. A dosage unit can be administrated once or several times a day, week or month. The delivery can also be continuously, for example by infusion or through a transdermal sustained release system.

"Aerial parts" as used in the present invention means the thin and/or medium leaves and/or stems of a respective plant material.

The provided plant materials can comprise further ingredients, for example compounds or other plant materials, as long as this ingredients do not inhibit or disturb the preparation of the pharmaceutically active extract and they are pharmaceutically acceptable.

A method in which the extracting step c) and the mixing step d) are conducted together in a single step, for example by extracting two of the first plant material, the second plant material and the third plant material or all three plant materials together, is also considered.

A person skilled in the art will be aware that the term "filtrating" in step e) of the inventive method does also comprise other methods allowing the separation of the clean extract from the solid constituents of the plant material, for example decantating, centrifugating, sedimentating, sieving, chromatographing, etc.

Preferably, the provided plant materials are cleaned and dried before applying to the inventive method. Preferably, the plant materials are dried in the dark for at least one day by avoiding any humidity preferably at a temperature above room temperature.

Room temperature will be understood by a person skilled in the art as a temperature in the range from about 18 to about 25°C.

Preferably, at least the first plant material and the second plant material are fragile after the drying step and before used in the inventive method.

Preferably, the extracting step is conducted in the dark.

The organic solvent used in the extraction step can also be a mixture of different organic solvents and/or water.

Preferably, the pharmaceutical composition according to the present invention comprises selenium, more preferably in a ratio of 1:1.000 to 1 to 1.000.000 by weight with regard to the weight of the pharmaceutically active extract.

Even preferred, the inventive pharmaceutical composition further comprises urea and/or phytoprotein in a ratio of 1:1.000 to 1:1.000.000 by weight with regard to the weight of the pharmaceutically active extract.

Further preferred, the inventive pharmaceutical composition further comprises at least one antioxidant, more preferably in an amount of 1:1.000 to 1:1.000.000 by weight with regard to the weight of the pharmaceutically active extract.

Most preferably, the antioxidant is beta-carotene.

It will be understood by those skilled in the art that various modifications and substitutions may be made to the invention as described above without departing from the spirit and scope of the invention. Accordingly, it is understood that the present invention has been described by way of illustration and not limitation. The advantages of the present invention will also be apparent by the following detailed description on the basis of the examples.

### EXAMPLE 1

### Mowing and drying plants

Thin and medium-sized leaves and stems of *Tanacetum Vulgare L.* and *Urtica Dioica L.* were mowed and transferred to warehouse in order to dry out. Medium leaves and stems were mowed by hand or sickle and washed after separation and cleaning. Washing the plants is recommended to remove probable contamination. The plants were transferred to warehouse for drying.

Drying was conducted as follows:

Aerial parts of the plants (tiny and medium leaves and stems) were spread on wooden shelves covered with hemp. The shelves consisted of 10-15 rows with 25 cm height each. Surface of the shelves was covered with hemp. The plants were spread on the shelves in one-layer. In order to prevent heat waste, the environment of the warehouse was isolated. The temperature of warehouse was about 40-45°C. The plants produce moisture during the procedure. Therefore, in order to remove this humidity, the warehouse had air conditioner. Plants were spread in one layer to avoid that the humidity from lower parts spoiled the upper part and vice versa.

**Note:** It is recommended to install an air vent in addition to heater (to heat the environment) in the warehouse.

**Light:** Plants should be dried in a dark room.

**Time:** Usually, plants are dried in 3-4 days.

### Satisfactory criteria of drying

Dried plant should be fragile.

The plants should remain green and should retain their color after drying.

### Cleaning and grinding

After drying, the plants were put in hemp bags and moved to drug production (extraction) area. They were kept there until the time of the extraction procedure. In order to prevent cross contamination (i.e. existence of other dried plants in bags), all dried plants should be taken out of the bags after cleaning put in boxes. *Urtica Dioica L.* and *Tanacetum Vulgare L.* were grinded. *Rosa Canina* fruits were separated for extraction procedure.

After being dried and grinded, plants were kept at a dry and dark place. Their quality for drug production was maintained for about 1-2 years.

### EXAMPLE 2

### Extraction

### 3.21 bottles with metal lids and plastic washers were used for extraction procedure.

To produce the pharmaceutically active extract, extraction procedure of the 3 plant materials was performed independently. The quantity of each plant and the weight ratios of the dried plants to alcohol were as follow:
1. The ratio of dried *Urtica Dioica L.* to alcohol: 700 g of dried *Urtica Dioica L.* and 2.45 liter of 96% ethanol were put in a 3.2 liter container. *Urtica Dioica L.* comprised dried and grinded leaves and medium and small stems. The plants were completely pressed and compacted.
2. The ratio of dried *Tanacetum Vulgare L.* to alcohol: 880 g of dried *Tanacetum Vulgare L.* and 2.0 liter of 96% ethanol were put in a 3.2 liter container. *Tanacetum Vulgare L.* comprised dried and grinded leaves with medium and small stems. The plants were completely pressed and compacted.
3. The ratio of dried *Rosa Canina L.* to alcohol: 850 g of dried *Rosa Canina* fruits and 2.36 liter of 96% ethanol were put in a 3.2 liter container. The plant contains dried fruit of *Rosa Canina* and care was taken not to grind these fruits.

After filling the containers with ethanol, they were closed and placed in incubator. Temperature of incubator was 42°C and the environment was dark. The containers remained under this conditions for about 25-30 days. As product, extracts were obtained. The extraction of *Urtica Dioica L.* is darker (green) than that of *Tanacetum Vulgare L. Rosa Canina* extract is of orange-red color.

During extraction, the containers stayed closed. The duration of extraction was 25-30 days. The plant material in the bottles turned colorless and the solution became dark green.

### EXAMPLE 3

### Mixing the extract

On the 30th day, the filtered red-orange extract of *Rosa Canina* was separated.

The containers containing *Urtica Dioica L.* and *Tanacetum Vulgare L.* were taken out of the incubator and 100ml of the *Rosa Canina* extract were added to each of them. The *Urtica Dioica L.* and *Tanacetum Vulgare L.* extracts were not filtered and still contained the herbal components. Afterwards, the containers containing *Urtica Dioica L.* and *Tanacetum Vulgare L.* extract with 100ml extract of *Rosa Canina* were pressed and shaken. 2 sets of containers were obtained, each containing the following component:
**First set:** containers containing non-filtered extract of *Urtica Dioica* + 100 ml of the extract of *Rosa Canina.*
**Second set:** containers containing non-filtered extract of *Tanacetum Vulgare* + 100 ml of the extract of *Rosa Canina*

The resulting extracts were filtered with 30 µm filter papers and put in 20 liter containers and mixed.

After filtering through 8, 3, and 0.22 µm, the extract was sterilized. Before using in capsules, the alcoholic extract exposed to electromagnetic radiation for about 3 minutes with a radiation of 11.5 V and 1 A and dried.

### EXAMPLE 4

To prepare a pharmaceutical composition, the pharmaceutically active extract prepared in Example 3 was mixed with suitable excipients, such as fillers and lubricants. The amounts of compounds which were used for preparing the pharmaceutical compositions are shown in Table 1. After achieving of the bulk material containing the extract, it was entered into size zero capsules by using a MG- 2 filling machine.

**Table 1 Complete drug formulation (percentage of main and complementary compounds)**

| **No.** | **ingredients** | **state of matter in the formulation** | **Percentage (w/v or w/w)** | **Role of the ingredients in the formulation** |
|---|---|---|---|---|
| **A. Main compounds** | | | | |
| 1 | The mixture of extracts | Extract | 100 mg/cap | anti-inflammatory, increase the neural signets, promote regeneration of peripheral nerves |
| 2 | Selenium | In complex with extract | 26.6 µg / cap | simulation of Immune system and anti oxidation effects |
| 3 | Urea and phytoprotein | In complex with extract | 40 µg / cap | Providing energy for cell simulation, normalizing blood flow to/from micro vessels |
| 4 | Beta-Carotene | In complex with extract | 16.106 µg / cap | Adjusting metabolism processes and anti-oxidant |

| **B. complementary compounds** | | | | |
|---|---|---|---|---|
| 5 | Talc | Powder | 15 mg / cap | Lubricant |
| 6 | Corn starch | Powder | 85mg /cap | Filler |
| 7 | Lactose | White crystalline powder | 300mg / cap | Filler, Binder |

### EXAMPLE 5

Preclinical toxicological investigations of the pharmaceutically active extract according to the present invention were done to study the toxicity of the extract in injection to BALB/C mice and Wistar rats. In the tested doses and schedules, the extract did not cause allergenic reaction. In the same way, no serious toxic properties of the extract were obtained. As a result of the toxicology studies, the extract was recommended as a save component for different phases of clinical trial.

Phase I clinical trial of the extract was conducted to determine the maximum tolerated dose and dose limited toxicities. The results indicated that the extract can be tolerated up to much higher dosis than considered for treatment.

Phase II clinical trial of the extract was conducted in 12 patients suffering from diabetic neuropathy. After six months of treatment about 70% of the patients had a great satisfaction as their problems were mainly removed. Based on the results of phase II of clinical trial, it was shown that the extract was safe. No important side effects following its administration were investigated. Following the satisfying results of phase II of clinical trial, the phase III of clinical trial was conducted.

Phase III experiments were performed as double blind randomized clinical trial (RCT) to investigate the efficiency of the inventive pharmaceutical composition.

The RCT was conducted on 150 patients suffering from peripheral diabetic neuropathy. The results based on interim analysis of 65 patients that completed the study. T-test and ANOVA and Tukey were applied for the comparison of the variables within the study groups. P-values lower than 0.05 were considered as significant.

### Results

From among 65 patients recruited in the study, 60 subjects remained until the end of the study. The mean age of the group, consisting of 18 males and 42 females was 55.4 ± 5.6 years. There were no significant differences between the mean weight and blood pleasure values of this three studied groups (inventive extract, 24 patients; gabapentin, 18 patients; and placebo, 18 patients). The mean main score, based on VAS measurement, was not statistically different between the three studied groups (p-value = 0.198). In the inventive extract and gabapentin group, however, significant difference was noted between the pain score measured at the base line and the 20^{th} week of treatment (p-value > 0.001).

Based on MNS criteria, there was a significant difference in the mean score the neurological symptoms and signs at the baseline in the 20^{th} week of treatment (p-value = 0.030). Turkey analysis revealed a significant difference between the inventive extract and placebo group (p-value = 0.037).

Comparison of the placebo and inventive extract group, based on MNS (Michigan Neuropathy Score), Total Neuropathy Score (TNS) and Neuropathy Symptom Score (NSS) criteria, demonstrated a significant difference in their score measured at the baseline and the 20^{th} week of treatment (p-value > 0.05). Conversely, a significant difference was absent in the neurotesiometer nerve conduction study within two groups (p-value > 0.05). A significant increase, however, was noted in the nerve conduction filler velocity in sensory, multiage it and peroneal nerves at the baseline and the 20^{th} week, based on Visual Analog Scale VAS, MNS, NSS and TNS measurements, of the treatment in the extract and gabapentin groups (p-value > 0.05).

The features disclosed in the foregoing description and in the claims may both separately and in any combination thereof be material for realizing the invention in diverse forms thereof.

## Claims

1. Method for preparing a pharmaceutically active extract comprising the steps:
a) providing a first plant material of *tanacetmn vulgare L.,* a second plant material of *urtica dioica L.,* and a third plant material of *rosa canina*;
b) optionally grinding the first plant material and the second plant material;
c) extracting the first plant material, the second plant material and the third plant material separate from each other by means of an organic solvent to obtain a first extract from the first plant material, a second extract from the second plant material and a third extract from the third plant material, wherein the first extract the second extract and the third extract can comprise at least parts of the respective plant material;
d) mixing at least part of the first extract, at least part of the second extract and at least part of the third extract to obtain an extract mixture; and
e) filtrating and optionally concentrating the extract mixture to obtain the pharmaceutically active extract.

2. Method according to claim 1, wherein the first plant material comprises the aerial parts of *tanacetum vulgare L.*

3. Method according to claim 1 or 2, wherein the second plant material comprises the aerial parts of *urtica dioica L.*

4. Method according to any of the preceding claims, wherein the third plant material comprises the fruits of *rosa canina.*

5. Method according to any of the preceding claims, wherein the organic solvent is at least one alcohol, preferably is ethanol.

6. Method according to any of the preceding claims, wherein the plant material:alcohol ratio in the extracting step c) is 5:1-1:20 by weight, preferably 1:1-1:5 by weight.

7. Method according to any of the preceding claims, wherein at least one of the steps a)-e) is carried out in the dark.

8. Method according to any of the preceding claims, wherein extracting is carried out in a temperature range from 20-100°C, preferably in a temperature range of 20-60°C, most preferably in a temperature range from 30-50°C.

9. Method according to any of the preceding claims, wherein extracting is conducted for 1-200 days, preferably for 7-70 days, most preferably for 10-40 days.

10. Method according to any of the preceding claims, wherein concentrating is concentrating to dryness.

11. Pharmaceutically active extract obtained by a method according to any of the preceding claims.

12. Pharmaceutical composition comprising the pharmaceutically active extract according to claim 11.

13. Pharmaceutical composition according to claim 12 further comprising selenium, urea and/or phytoprotein and/or an antioxidant as well as optionally further excipients such as lubricants, fillers and/or binders.

14. Pharmaceutically active extract according to claim 11 and/or pharmaceutical composition according to claim 12 or 13 for use in the treatment of diabetic neuropathy and/or the symptoms of diabetic neuropathy.

15. Pharmaceutically active extract according to claim 14, wherein the diabetic neuropathy is peripheral diabetic neuropathy.
